(19) Europäisches Patentamt
European Patent Office
Office européen des brevets

(11) EP 2 949 385 A1

(12) EUROPEAN PATENT APPLICATION
published in accordance with Art. 153(4) EPC

(43) Date of publication:
**02.12.2015 Bulletin 2015/49**

(21) Application number: **14743074.8**

(22) Date of filing: **20.01.2014**

(51) Int Cl.:
***B01D 71/56*** *(2006.01)* ***B01D 61/36*** *(2006.01)*
***B01D 69/10*** *(2006.01)* ***B01D 69/12*** *(2006.01)*
***B01D 71/52*** *(2006.01)* ***B32B 27/34*** *(2006.01)*
***C02F 1/44*** *(2006.01)* ***C07C 37/70*** *(2006.01)*
***C07C 39/04*** *(2006.01)*

(86) International application number:
**PCT/JP2014/051007**

(87) International publication number:
**WO 2014/115687 (31.07.2014 Gazette 2014/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **25.01.2013 JP 2013012301**
**25.06.2013 JP 2013133154**

(71) Applicant: **Sumitomo Bakelite Company Limited**
**Shinagawa-ku**
**Tokyo 140-0002 (JP)**

(72) Inventors:
- **HASHIMOTO, Ryoma**
  **Tokyo 140-0002 (JP)**
- **MIYAUCHI, Hiroyuki**
  **Tokyo 140-0002 (JP)**

(74) Representative: **Müller-Boré & Partner**
**Patentanwälte PartG mbB**
**Friedenheimer Brücke 21**
**80639 München (DE)**

# (54) PERVAPORATION MEMBRANE AND METHOD FOR CONCENTRATING PHENOL

(57) A pervaporation membrane having superior durability and a phenol concentrating method which can efficiently concentrate phenols with the pervaporation membrane are provided. A pervaporation separation apparatus 100 includes a treatment target liquid tank 110, a pervaporation membrane module 120, a permeated material recovering tank 130 and a non-permeated material storage tank 140. The pervaporation membrane module 120 includes a pervaporation membrane 1 having a porous support body and an enveloping layer formed of a polyamide-containing resin. The phenol concentrating method of the present invention includes a pretreatment process for pretreating phenol aqueous solution stored in the treatment target liquid tank 110, a pervaporation process for treating the phenol aqueous solution in the pervaporation membrane module 120 with a pervaporation method and a condensation process for condensing a permeated material permeating across the pervaporation membrane 1 to obtain concentrated phenol aqueous solution.

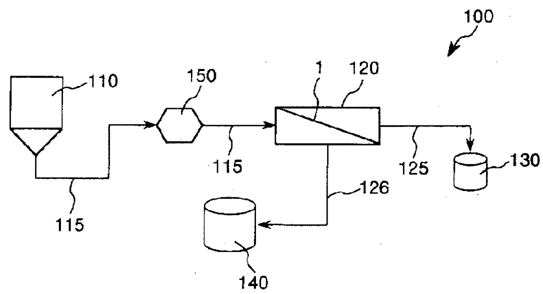


FIG.1



Printed by Jouve, 75001 PARIS (FR)

## Description

### TECHNICAL FIELD

The present invention relates to a pervaporation membrane and a method for concentrating phenols.

### BACKGROUND ART

A phenol resin is a representative thermosetting resin and used in many fields for parts, insulating bodies or the like which are required to have heat resistance.

When the phenol resin is produced, waste liquid containing a phenolic compound is produced. Since the phenolic compound has been raised as one of causative substances causing water pollution of a river or the like, a concentration (level) of the phenolic compound in the waste liquid to be discharged into a river or the like from an industrial plant is stringently regulated by laws and regulations or the like.

Under this circumstance, it has been developed that a method for removing the phenolic compound from the waste liquid containing the phenolic compound to decrease the concentration of the phenolic compound in the waste liquid for reducing an environmental burden. Patent document 1 discloses a treatment method for phenol-containing waste liquid. The treatment method disclosed in the patent document 1 includes supplying the phenol-containing waste liquid into a separating device having a pervaporation membrane, and separating and recovering phenols from permeated liquid at a depressurized side.

In such a treatment method, the pervaporation membrane is used for preferentially osmosing and transferring the phenols across the pervaporation membrane to concentrate the phenols. By preferentially osmosing and transferring the phenols across the pervaporation membrane, it is possible to decrease the concentration of the phenols in the waste liquid which does not permeate across the pervaporation membrane. However, deterioration of the pervaporation membrane causes when the phenols permeate across the pervaporation membrane. Accumulation of the deterioration of the pervaporation membrane results in a breaking of the pervaporation membrane, deterioration of a phenol permeability of the pervaporation membrane or the like. These cause a problem in that a concentration efficiency of the phenols gradually decreases with time.

### RELATED ART

### PATENT DOUMENT

Patent document 1: JP H06-296831A

### SUMMARY OF INVENTION

### PROBLEMS TO BE SOLVED BY THE INVENTION

It is an object of the present invention to provide a pervaporation membrane having superior durability and a phenol concentrating method for efficiently concentrating phenols with the pervaporation membrane.

### MEANS FOR SOLVING PROBLEMS

The above objection is achieved by the present invention including the following features (1) to (7).

(1) A pervaporation membrane to be used for concentrating phenols in liquid containing the phenols, water and an inorganic ion with a pervaporation method, the pervaporation membrane comprising:

a porous support body; and
an enveloping layer provided so as to envelop the porous support body, the enveloping layer formed of a polyamide-containing resin.

(2) The pervaporation membrane according to the above (1), wherein the polyamide-containing resin is a copolymer containing a polyamide segment.

(3) The pervaporation membrane according to the above (2), wherein the copolymer further contains a polyether segment.

(4) The pervaporation membrane according to the above (3), wherein the copolymer is a block copolymer containing 10 to 90 mole% of the polyamide segment.

(5) The pervaporation membrane according to any one of the above (1) to (4), wherein the support body is a woven cloth or a nonwoven cloth.

(6) The pervaporation membrane according to the above (5), wherein the support body is constituted of a glass fiber.

(7) A phenol concentrating method comprising:

concentrating phenols in liquid containing the phenols and water with a pervaporation method using a pervaporation membrane defined in any one of the above (1) to (6).

## EFFECTS OF INVENTION

According to the present invention, it is possible to obtain the pervaporation membrane having superior durability for separating the phenols.
In addition, according to the present invention, by using the aforementioned pervaporation membrane, it is possible to efficiently separate and concentrate the phenols without frequently exchanging the pervaporation membrane.

## BRIEF DESCRIPTION OF DRAWINGS

Fig. 1 is a view showing one example of a pervaporation separation apparatus having a pervaporation membrane of the present invention.
Fig. 2 is an enlarged view showing a pervaporation membrane module in the pervaporation separation apparatus shown in Fig. 1.
Fig. 3 is a partially-enlarged view of the pervaporation membrane shown in Fig. 2.
Fig. 4(a) is a process chart for a phenol concentrating method according to an embodiment of the present invention.
Fig. 4(b) is a view for explaining changing of a treated material in the phenol concentrating method according to the embodiment of the present invention.

## MODE FOR CARRYING OUT THE INVENTION

Hereinafter, a pervaporation membrane and a phenol concentrating method of the present invention will be described in detail with reference to preferred embodiments shown in the accompanying drawings.

<Pervaporation separation apparatus>

The phenol concentrating method of the present invention is a method for concentrating phenols in liquid containing the phenols, water and an inorganic ion with a pervaporation method using the pervaporation membrane of the present invention. The words of "pervaporation method" means a method for concentrating a solute in a state that a liquid phase and a vapor phase are separated by a pervaporation membrane. In this method, the solute is preferentially transferred across the pervaporation membrane by using partial pressure of the solute on the side of the liquid phase as driving force to concentrate the solute. Such a pervaporation method is carried out with a pervaporation separation apparatus having the pervaporation membrane.
Fig. 1 is a view showing one example of the pervaporation separation apparatus having the pervaporation membrane of the present invention. Fig. 2 is an enlarged view showing a pervaporation membrane module in the pervaporation separation apparatus shown in Fig. 1. Fig. 3 is a partially-enlarged view of the pervaporation membrane shown in Fig. 2.
A pervaporation separation apparatus 100 shown in Fig. 1 includes a treatment target liquid tank 110, a pervaporation membrane module 120, a permeated material recovering tank 130 and a non-permeated material storage tank 140.
Among them, a supply conduit 115 connects between the treatment target liquid tank 110 and the pervaporation membrane module 120. Further, a pretreatment module 150 is provided in the middle of the supply conduit 115. Examples of the pretreatment module 150 include a module for a sedimentation treatment with a flocculant, a module for a filtration treatment, a module for a reverse osmosis membrane treatment, a module for an azeotropic treatment and a module

for a distillation treatment.

On the other hand, a permeation conduit 125 connects between a permeation side (secondary side) of the pervaporation membrane module 120 and the permeated material recovering tank 130. Further, a discharge conduit 126 connects between a supply side (primary side) of the pervaporation membrane module 120 and the non-permeated material storage tank 140.

As shown in Fig. 2, the pervaporation membrane module 120 has a housing 121 and a pervaporation membrane 1 provided so as to separate an internal space of the housing 121. In the internal space of the housing 121, the primary side of the pervaporation membrane 1 is a supply side space 122 and the secondary side of the pervaporation membrane 1 is a permeation side space 123.

Examples of treatment target liquid to be treated in the pervaporation separation apparatus 100 include liquid (solution or dispersion liquid) in which organic substances are dissolved or dispersed. In the pervaporation separation apparatus 100, the organic substances are preferentially separated from the treatment target liquid, with a pervaporation method. Due to this preferential separation of the organic substances, a "concentration" for increasing a concentration of the organic substances in the treatment target liquid can be carried out. This makes the treatment target liquid after being concentrated easier for using as an industrial raw material or the like, and thereby improving utility value of the treatment target liquid. Further, in the case where the treatment target liquid contains toxic substances, it is possible to carry out a volume reduction of the toxic substances due to the concentration to improve an efficiency of a waste liquid treatment or a detoxifying treatment.

In this specification, although description will be especially given to the concentration of phenols as a representative example, examples of the organic substances which can be concentrated in the pervaporation separation apparatus 100 include alcohols such as methanol, ethanol, isopropyl alcohol and butanol; pyridine; chloroform and tetrachloroethylene, in addition to the phenols. Further, the pervaporation separation apparatus 100 can concentrate, for example, a liquid crystal material for a display such as n-butylbenzene; a wide variety of agricultural chemicals such as 1,2-dibromo-3-chloropropane, 2-butylphenylmethylcarbamate and bendiocarb; a plasticizing agent such as diethyl phthalate; coplanar PCB and dibenzo-p-dioxine which are categorized as an endocrine disrupting chemical (environmental hormone).

On the other hand, examples of a solvent for dissolving the organic substances or a dispersion medium for dispersing the organic substances include water.

The treatment target liquid may contain other materials than the organic substances and the water such as an inorganic salt and a dissolved material thereof (inorganic ionic impurity). Examples of the other materials include an inorganic ion such as sodium ion, potassium ion, sulphate ion, phosphate ion and ammonium ion.

Next, description will be given to a working example of the pervaporation separation apparatus 100. In this section, description will be given to an exemplary case where water (aqueous solution) in which the organic substances are dissolved is used as the treatment target liquid.

First, the permeation side of the pervaporation membrane module 120 is depressurized with a depressurizing pump (not shown) connected to the permeation conduit 125.

Next, the treatment target liquid stored in the treatment target liquid tank 110 is transferred into the pretreatment module 150 through the supply conduit 115 to pretreat the treatment target liquid in the pretreatment module 150. In the pretreatment, foreign substances in the treatment target liquid are removed and a volume reduction of the treatment target liquid is carried out.

Next, the treatment target liquid is transferred into the supply side space 122 of the pervaporation membrane module 120 through the supply conduit 115. In the pervaporation membrane module 120, the organic substances in the treatment target liquid are preferentially transferred across the pervaporation membrane 1 by depressurizing the permeation side space 123 as described above. As a result, the concentration of the organic substances in the treatment target liquid on the permeation side increases and the treatment target liquid on the permeation side is concentrated.

The treatment target liquid thus concentrated is transferred into the permeated material recovering tank 130 through the permeation conduit 125 and recovered. On the other hand, the treatment target liquid remaining in the supply side space 122 of the pervaporation membrane module 120 is transferred into the non-permeated material storage tank 140 through the discharge conduit 126.

The non-permeated material storage tank 140 and the treatment target liquid tank 110 may be connected through a conduit to transfer the treatment target liquid stored in the non-permeated material storage tank 140 back to the treatment target liquid tank 110 again, as needed. With this configuration, it is possible to repeatedly transfer the same treatment target liquid into the pervaporation membrane module 120. This makes it possible to more reliably separate the organic substances which cannot be separated in just one treatment, and thereby improving a recovery rate of the organic substances.

<Pervaporation membrane>

Next, description will be given to an embodiment of the pervaporation membrane of the present invention.

The pervaporation membrane 1 shown in Fig. 2 is provided so as to separate the internal space of the housing 121 of the pervaporation membrane module 120 into the supply side space 122 and the permeation side space 123 as described above. A form of the pervaporation membrane 1 is not limited to a specific form. For example, a highly dense membrane in the form of a flat membrane form, a hollow fiber form, a tube-shaped form, a bag-shaped form or a spiral form may be used as the pervaporation membrane 1.

The pervaporation membrane 1 has a porous support body 11 and an enveloping layer 12 provided so as to envelop the support body 11. The enveloping layer 12 is formed of a polyamide-containing resin. In other words, the support body 11 is embedded in the enveloping layer 12. Since the enveloping layer 12 contained in such a pervaporation membrane 1 has superior affinity with respect to organic substances, the enveloping layer 12 allows the organic substances in the treatment target liquid to preferentially permeate. Thus, it is possible to efficiently separate and concentrate the organic substances in the treatment target liquid. Further, since the pervaporation membrane 1 has the support body 11, it is possible to reinforce the enveloping layer 12 and improve a mechanical property of the pervaporation membrane 1 without significantly deteriorating the affinity of the enveloping layer 12 with respect to the organic substances. With this configuration, it is possible to obtain the pervaporation membrane 1 having both a separation property for the organic substances and durability.

In this regard, an entire surface of the support body 11 may not be enveloped by the enveloping layer 12 and a part of the surface of the support body 11 may be exposed. Even in the case where the part of the surface of the support body 11 is exposed, the support body 11 can reinforce the enveloping layer 12. Thus, it is possible to provide the effect of improving the mechanical property of the pervaporation membrane 1.

(Enveloping layer)

The enveloping layer 12 is a highly dense layer provided so as to envelop the support body 11. This enveloping layer 12 is formed of the polyamide-containing resin.

As the polyamide-containing resin, a polymer having a repeating unit containing an amide bond can be used. The kind of such a polymer is not particularly limited to a specific kind. Although detailed reasons are unknown, it is considered that the enveloping layer 12 formed of the polyamide-containing resin can efficiently absorb and osmose the organic substances (in particular, phenols, the same applies hereafter) to allow the organic substances to preferentially permeate across the enveloping layer 12 because the amide bond has high affinity with respect to the organic substances. Thus, the pervaporation membrane 1 including such an enveloping layer 12 especially has a superior separation property.

For example, the polyamide-containing resin may be a polyamide resin such as nylon 6, nylon 66 and nylon 12; a polymer alloy or a polymer blend containing one or more of these polyamide resins. Especially, the polyamide-containing resin is preferably a copolymer having the repeating unit containing the amide bond. In particular, it is preferably to use a copolymer containing a polyamide segment constituted of the repeating unit containing the amide bond and another segment which can copolymerize with this polyamide segment as such a copolymer. In the pervaporation membrane 1 constituted of such a copolymer, it is possible to provide the affinity with respect to the organic substances in the treatment target liquid and improve the mechanical property of the pervaporation membrane 1 by the polyamide segment mainly. On the other hand, by appropriately selecting the other segment, it is possible to ensure permeability for the organic substances without deteriorating the affinity with respect to the organic substances in the treatment target liquid. Thus, by using the copolymer containing the polyamide segment and the other segment appropriately selected, it is possible to improve the superior permeability and the superior affinity with respect to the organic substances in the treatment target liquid and ensure the high durability of the pervaporation membrane 1.

Especially, in the pervaporation method, the organic substances osmose into the pervaporation membrane 1 and permeate across the pervaporation membrane 1 with swelling the pervaporation membrane 1. However, by repeating the swelling of the pervaporation membrane 1, there is a case where a breaking of the pervaporation membrane 1 occurs and the separation property deteriorates due to the deterioration of the pervaporation membrane 1 caused by the swelling. In contrast, in the pervaporation membrane 1 containing the copolymer described above, since the deterioration of the mechanical property caused by the swelling of the pervaporation membrane 1 is suppressed by the polyamide segment and the permeability for the organic substances is improved by the other segment, it is considered that the deterioration caused by the osmosis and the swelling is suppressed even if the organic substances osmose into the pervaporation membrane 1 and the pervaporation membrane 1 swells, and thereby improving the durability of the pervaporation membrane 1.

Examples of the other segment which can copolymerize with the polyamide segment include a polyether segment constituted of a repeating unit containing an ether bond, a polyester segment constituted of a repeating unit containing an ester bond, a polyethylene segment constituted of a repeating unit containing an ethylene structure, a

polypropylene segment constituted of a repeating unit containing a propylene structure and a polysiloxane segment constituted of a repeating unit containing a siloxane bond. These segments may be used singly or in combination of two or more of them.

Among them, the other segment which can copolymerize with the polyamide segment preferably contains the polyether segment. The polyether segment can easily copolymerize with the polyamide segment and has higher flexibility than that of the polyamide segment. Thus, even if large external force is added to the pervaporation membrane 1, it is possible to prevent the pervaporation membrane 1 from being easily broken. Further, the polyether segment has low affinity with respect to a wide variety of organic substances. Thus, it is possible to suppress significant deterioration of the pervaporation membrane 1 caused by the swelling and the osmosis of the organic substances in the pervaporation membrane 1. As a result, it is possible to obtain the pervaporation membrane 1 more highly striking the balance between the separation property for the organic substances and the durability.

Further, the polyether segment has a hydrophilic property. Thus, in the case where the treatment target liquid is aqueous liquid, the pervaporation membrane 1 containing the polyether segment has a high contact property with respect to the treatment target liquid. On the other hand, the polyamide segment described above has a lipophilic property. Thus, whereas the aqueous treatment target liquid contacts with the pervaporation membrane 1 smoothly, the organic substances in the treatment target liquid efficiently osmose into a lipophilic part of the pervaporation membrane 1. As a result, it is possible to further improve the separation property for the organic substances. From the viewpoint described above, it is preferably to use a segment having a hydrophilic property as the other segment which can copolymerize with the polyamide segment. The following formula (1) is one example of a structural formula of the polyamide segment.

[Formula 1]

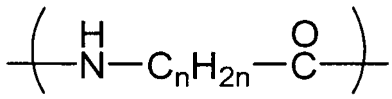

(1)

In the formula (1), "n" represents an integer of 1 to 8.

The following formula (2) is one example of a structural formula of the polyether segment.

[Formula 2]

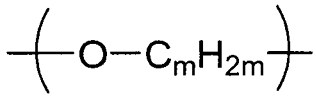

(2)

In the formula (2), "m" represents an integer of 1 to 8.

The copolymer containing the polyamide segment and the other segment preferably contains 10 to 90 mole% of the polyamide segment, more preferably contains 20 to 80 mole% of the polyamide segment, more preferably contains 30 to 75 mole% of the polyamide segment, and even more preferably contains 45 to 75 mole% of the polyamide segment. The copolymer containing the polyamide segment in the above rate can provide the high affinity with respect to the organic substances and the high mechanical property imparted to the pervaporation membrane 1 which are caused by the polyamide segment mainly and the high permeability with respect to organic components which is caused by the other segment mainly with little losing both effects caused by the polyamide segment and the other segment. Further, by setting the rate of each segment to fall within the above range, it is possible to suppress the deterioration of the separation property of the enveloping layer 12 caused by embedding the support body 11 into the enveloping layer 12. Namely, when the support body 11 is embedded into the enveloping layer 12, the support body 11 is located on a permeation path for substances permeating across the pervaporation membrane 1. Thus, an apparent effective area of the pervaporation membrane 1 decreases. Due to the decrease of the apparent effective area, there is a risk that the separation property deteriorates. However, by setting the rate of each segment to fall within the above range, it is possible to facilitate the absorption and the osmosis of the organic substances as much as possible by the polyamide segment, and thereby avoiding the influence caused by the support body 11. In addition, by embedding the support body 11 into the enveloping layer 12, it is possible to improve the mechanical property of the pervaporation membrane 1. Thus, for example, even if the copolymer containing the polyamide segment and the other segment swells, it is possible to suppress that the pervaporation membrane 1 deteriorates to a level in which the pervaporation membrane 1 loses the function of

a barrier. Thus, by setting the rate of the polyamide segment and the other segment to fall within the above range in addition to by providing the support body 11, it is possible to obtain the pervaporation membrane 1 reliably striking the balance between the separation property for the organic substances and the durability. Especially, in the case of using the polyether segment as the other segment, the permeability for the organic substances is improved. Thus, it is considered that a stay time of the organic substances in the pervaporation membrane 1 reduces and the swelling become unlikely to occur, and thereby further improving the separation property.

The copolymer containing the polyamide segment may be one of a block copolymer, a random copolymer and an alternating copolymer. Among them, it is preferable that the copolymer is the block copolymer. With this, it is possible to provide both the property caused by the polyamide segment and the property caused by the other segment without canceling the properties with each other, and thereby obtaining the pervaporation membrane 1 reliably striking the balance between the separation property for the organic substances and the durability.

Examples of the polymer alloy or the polymer blend containing the polyamide resin include a polymer obtained by adding polyethylene, polypropylene, polystyrene, polyacrylate, polyacrylamide, polydimethylsiloxane, polyvinylpyrrolidone, polyurethane or the like into the polyamide resin. These polymers may be used singly or in combination of two or more of them. It is considered that these polymers can contribute to reinforcing of the polyamide resin without deteriorating the absorption and the osmosis of the organic substances due to the polyamide resin. Thus, from the viewpoint of striking the balance between the separation property and the durability of the pervaporation membrane 1, the polymer alloy or the polymer blend containing these resin components in addition to the polyamide resin is useful. Further, as mentioned below, it is possible to further improve the durability by irradiating the pervaporation membrane 1 with an electron beam. It is considered that this is because a cross-link is generated between these resins and the polyamide by the irradiation of the electron beam. Especially, among these resins, the polyethylene is preferably used from the viewpoints of chemical resistance, long-term stability and mechanical strength.

Although an average thickness of the pervaporation membrane 1 described above is not limited to a specific value as long as the pervaporation membrane 1 has mechanical strength required for separating a liquid phase and a vapor phase, the average thickness of the pervaporation membrane 1 is preferably in the range of about 10 to 500 μm, and more preferably in the range of about 20 to 400 μm. By setting the average thickness of the pervaporation membrane 1 to fall within the above range, it is possible to reliably separate the supply side space 122 and the permeation side space 123 and sufficiently ensure the separation property for the organic substances.

A Shore D hardness of a hardened material of the polyamide-containing resin is preferably in the range of about 15 to 85, and more preferably in the range of about 20 to 75. By setting the Shore D hardness to fall within the above range, it is possible to suppress the pervaporation membrane 1 from being broken or stretched to a breaking point at the time of the separation in the pervaporation method and ensure a sufficient separation property. The Shore D hardness can be measured with a measuring method defined by ISO 868.

Further, a bending elastic modulus of the hardened material of the polyamide-containing resin is preferably in the range of about 10 to 550 MPa, more preferably in the range of about 25 to 400 MPa, and even more preferably in the range of about 40 to 300 MPa. By setting the bending elastic modulus to fall within the above range, it is possible to prevent the pervaporation membrane 1 from being broken or stretched to the breaking point and ensure the sufficient separation property. The bending elastic modulus of the polyamide-containing resin can be measured with a measuring method defined by ISO 178. In the measuring method, a thickness of a test piece is set to 100 μm, a width of the test piece is set to 10 mm, and a distance between support points is set to 50 mm.

Further, a melting point of the hardened material of the polyamide-containing resin is preferably in the range of about 110 to 185°C, and more preferably in the range of about 130 to 175°C. By setting the melting point of the polyamide-containing resin to fall within the above range, it is possible to obtain the pervaporation membrane 1 more highly striking the balance between the separation property for the organic substances and the durability. The melting point of the polyamide-containing resin can be measured by a measuring method defined by ASTM D3418.

Further, a heat distortion temperature of the hardened material of the polyamide-containing resin is preferably in the range of about 35 to 140°C, and more preferably in the range of about 40 to 130°C. By setting the heat distortion temperature of the polyamide-containing resin to fall within the above range, it is possible to suppress the deterioration of the separation property at the time of the separation in the pervaporation method. Namely, if the heat distortion temperature is lower than the above lower limit, there is a risk that the mechanical strength of the pervaporation membrane 1 deteriorates at the time of raising a temperature of the treatment target liquid for facilitating the pervaporation. On the other hand, if the heat distortion temperature is higher than the above upper limit, there is a risk that the pervaporation membrane 1 becomes likely to be influenced by the swelling of the organic substances and the durability of the pervaporation membrane 1 deteriorates. The heat distortion temperature of the polyamide-containing resin can be measured by a measuring method defined by ISO 75. In the measuring method, pressure added to a test piece is set to 0.46 MPa.

The copolymer containing the polyamide segment and the polyether segment can be produced with the existing synthesis method. Examples of the synthesis method include a method of copolymerizing a monomer for forming the polyamide such as lactams, ω-amino acids, diamine and dicarboxylic acid in the presence of polyalkylene ether having

an amino group at its terminal or an organic acid salt thereof; a method of copolymerizing the monomer for forming the polyamide in the presence of polyalkylene ether having a carboxyl group at its terminal or an organic amine salt thereof; and a method of copolymerizing the monomer for forming the polyamide in the presence of polyalkylene ether having the amino group, the carboxyl group or both the amino group and the carboxyl group at its terminal.

In the forming of the pervaporation membrane 1, a molding tool having a predetermined concave-convex shape may be pressed onto a surface of the pervaporation membrane 1 after forming a film of the polyamide-containing resin to transfer the concave-convex shape of the molding tool onto the surface of the pervaporation membrane 1. By transferring the concave-convex shape of the molding tool onto the surface of the pervaporation membrane 1, it is possible to increase a surface area of the pervaporation membrane 1, and thereby improving a permeation flux of the pervaporation membrane 1.

(Support body)

The pervaporation membrane 1 according to this embodiment includes the support body 11. The support body 11 is provided in the pervaporation membrane 1 or on the surface of the pervaporation membrane 1 to reinforce a membrane structure and improve the mechanical strength of the pervaporation membrane 1 as a whole.

The pervaporation membrane 1 shown in Fig. 3(a) includes the aforementioned enveloping layer 12 and the support body 11 provided so as to be embedded in the enveloping layer 12.

Examples of a form of the support body 11 include a cloth such as a non-woven cloth and a woven cloth, a punching material and a net material. Since each of these forms has a plurality of openings, each of these forms can be considered as a porous body. These openings on the porous body serve as paths for the organic substances. Further, the enveloping layer 12 is provided so as to infill (close) the openings. Thus, the organic substances osmose into the enveloping layer 12 to permeate across the pervaporation membrane 1.

A type of weave of the woven cloth is not particularly limited to a specific type. Examples of the type of weave of the woven cloth include a plain weave, a basket weave, a sateen weave and a twill weave.

Examples of a constituent material for the support body 11 include an inorganic fiber such as a glass fiber, a ceramic fiber, a carbon fiber and a metallic fiber; and an organic fiber such as a synthetic fiber (for example, an acrylic fiber, a nylon fiber, an aramid fiber, a polyester fiber, a polyethylene fiber, a polypropylene fiber and a rayon fiber) and a natural fiber (for example, linen, cotton, kenaf and jute). These fibers may be used singly or in combination of two or more of them as a composite fiber. Among them, the support body 11 formed of the glass fiber or the organic fiber is preferably used and the support body 11 formed of the glass fiber is more preferably used. Since the support body 11 formed of the above fiber has both superior flexibility and a superior mechanical property, the support body 11 is useful for supporting the pervaporation membrane 1 to which pressure is added over the long term. Namely, since the support body 11 formed of the above fiber is unlikely to be broken or pierced even if the pressure is added to the support body 11, it is possible to obtain the pervaporation membrane 1 having superior durability.

Among the above organic fibers, the organic fiber containing at least one of the nylon fiber and the aramid fiber is preferably used. Since these organic fibers contain an amide bond in their constituent materials, it is possible to obtain the support body 11 having an especially high adhesion property with respect to the poly-amide containing resin by using at least one of these organic fibers. Thus, gaps or holes are unlikely to generate in the pervaporation membrane 1 even if the pervaporation membrane 1 contains the support body 11, and thereby obtaining the pervaporation membrane 1 which can reliably suppress liquid leakage. In this regard, this effect can be also provided by using not only the nylon fiber and the aramid fiber but also another fiber containing the amide bond.

An occupancy rate (volume fraction) of the support body 11 in the pervaporation membrane 1 is not particularly limited to a specific value, but is preferably in the range of about 20 to 90 percent by volume, and more preferably in the range of about 30 to 80 percent by volume. By setting the occupancy rate to fall within the above range, it is possible to further improve the mechanical property of the pervaporation membrane 1 without deteriorating the separation property due to the polyamide-containing resin.

On the other hand, when the pervaporation membrane 1 is viewed (projected) in the planar view, an occupancy rate (area fraction) of the support body 11 is not particularly limited to a specific value, but is preferably in the range of about 10 to 95%, and more preferably in the range of about 20 to 90%. By setting the occupancy rate in the planar view to fall within the above range, it is possible to ensure a sufficient mechanical property and prevent the separation property of the pervaporation membrane 1 from being significantly deteriorated by the support body 11. In other words, it is possible to optimize the balance between the mechanical property and an opening ratio of the pervaporation membrane 1 to strike the balance between these properties.

The pervaporation membrane 1 including the support body 11 can be obtained through a process in that the aforementioned polyamide-containing resin is impregnated into the support body 11, casted and then pressured by a roller or the like as needed. In this process, the polyamide-containing resin is used in a state that the polyamide-containing resin is melted or diluted with an organic solvent as needed. For casting the polyamide-containing resin, a coating

apparatus such as a doctor blade is preferably used.

A surface treatment for improving the adhesion property with respect to the polyamide-containing resin may be applied to the surface of the support body 11 as needed. Examples of such a surface treatment include a coupling agent treatment and a plasma treatment.

Further, since the pervaporation membrane 1 includes the support body 11 therein, a concave-convex shape derived from the form of the support body 11 is likely to generate on the surface of the pervaporation membrane 1 (the surface of the enveloping layer 12). Due to the generation of such a concave-convex shape on the surface of the pervaporation membrane 1, the surface area of the pervaporation membrane 1 increases. As a result, the organic substances become likely to osmose into the pervaporation membrane 1, and thereby improving the separation property. On the other hand, depending on a level of the concave-convex shape, there is a risk that a breaking starting from a concave portion or the like becomes likely to occur in the pervaporation membrane 1. From this viewpoint, regarding a surface roughness of the pervaporation membrane 1, an arithmetic mean roughness Ra of a surface facing to the supply side space 122 (a surface on a side contacting with liquid) is preferably in the range of about 0.005 to 40 $\mu$m, and more preferably in the range of about 0.01 to 30 $\mu$m. On the other hand, an arithmetic mean roughness Ra of a surface facing to the permeation side space 123 (a surface on a desorption side) is preferably in the range of about 0.005 to 80 $\mu$m, and more preferably in the range of about 0.01 to 70 $\mu$m. By setting the surface roughness of each surface to fall within the above range, it is possible to sufficiently improve the separation property for the organic substances. In addition to this effect, it is possible to prevent the mechanical property of the pervaporation membrane 1 from deteriorating and, for example, suppress a breaking or the like of the pervaporation membrane 1.

Further, in the pervaporation membrane 1, it is preferable that the arithmetic mean roughness Ra of the surface facing to the permeation side space 123 is larger than the arithmetic mean roughness Ra of the surface facing to the supply side space 122. In particular, the arithmetic mean roughness Ra of the surface facing to the permeation side space 123 is preferably about 1.01 to 10000 times larger, and more preferably about 1.05 to 5000 times larger than the arithmetic mean roughness Ra of the surface facing to the supply side space 122. By setting the surface roughness of each surface in this manner, it is possible to individually optimize an osmosis efficiency for the organic substances on the surface facing to the supply side space 122 and a desorption efficiency for the organic substances on the surface facing to the permeation side space 123. Namely, since osmosis speed is rate-limited by desorption speed, by setting the surface roughness of each surface so that the desorption speed is moderately higher than the osmosis speed, it is possible to individually optimize the osmosis efficiency and the desorption efficiency and suppress the deterioration of the mechanical property.

For forming the concave-convex shape derived from the form of the support body 11, the support body 11 and the enveloping layer 12 may be pressed by a pressing member including a contacting surface having relatively high flexibility at the time of producing the pervaporation membrane 1. With this process, the shape derived from the support body 11 is likely to be reflected onto the surface of the enveloping layer 12. Examples of the contacting surface having the relatively high flexibility include a surface of a member formed of a rubber material such as a silicone rubber and a urethane rubber or a wide variety of elastomer materials.

Fig. 3(b) is a cross-sectional view showing another structural example of the pervaporation membrane 1 shown in Fig. 3(a). The pervaporation membrane 1 shown in Fig. 3(b) has the same configuration as the pervaporation membrane 1 shown in Fig. 3(a) except that the pervaporation membrane 1 shown in Fig. 3(b) includes the enveloping layer 12 and the support body 11 laminated on one surface of the enveloping layer 12. The pervaporation membrane 1 having such a configuration can be formed with, for example, a method of putting the enveloping layer 12 which is in an unhardened state or an un-solidification state on one surface of the support body 11 and then pressing the enveloping layer 12 onto the support body 11 with heating.

A plurality of the enveloping layers 12 and the support bodies 11 may be provided in one pervaporation membrane 1 as needed. For example, two enveloping layers 12 may be laminated so as to provide one support body 11 between them. Alternatively, three enveloping layers 12 and two support bodies 11 may be alternately laminated so as to be tucked.

In general, the concentration of the organic substances in the treatment target liquid to be treated in the pervaporation separation apparatus 100 is very low. Thus, it is expected to concentrate the organic substances to make them industrially applicable and carry out the volume reduction of the treatment target liquid, to which a disposal treatment or a detoxifying treatment is applied, with the pervaporation method using the pervaporation membrane according to the present invention.

For example, in the case where the treatment target liquid is phenol aqueous solution, a concentration of the phenol aqueous solution is generally a low concentration of 1 to 2 percent by mass. Thus, the phenol aqueous solution having such a low concentration is not suitable for industrial application (for example, application as a phenol raw material). Further, in most cases, the treatment target liquid is waste liquid from an industrial plant or the like, thus the treatment target liquid contains an impurity such as an inorganic salt and a polymer in addition to the phenols and water. Furthermore, for industrially applying the phenol aqueous solution, it is generally required that the phenol concentration

should be equal to or higher than 70 percent by mass. Thus, it is necessary to apply a separating and concentrating method such as a pervaporation method.

Generally, a zeolite membrane is known as a pervaporation membrane. An A-type zeolite membrane being put to practical use is a porous membrane having water permeability and has a function of allowing water in the phenol aqueous solution to preferentially permeate unlike the pervaporation membrane according to the present invention (the pervaporation membrane according to the present invention has the function of allowing the organic substances in the phenol aqueous solution to preferentially permeate). However, if the phenol aqueous solution contains the polymer or the like in addition to the phenols and the water as described above, the polymer causes a blockage of a membrane surface. As a result, the separation property deteriorates. Further, an ion exchanging between the inorganic salt contained in the phenol aqueous solution and a zeolite membrane surface occurs. This also results in the deterioration of the separation property. In addition, in the case where the phenol concentration is low, an amount of water permeating across the zeolite membrane increases in inverse proportional to the phenol concentration. This results in significant deterioration of an energy efficiency. Even in the case of using a polymer membrane formed of, for example, a polyvinyl alcohol other than the zeolite membrane, durability is low and the energy efficiency is also low if the phenol concentration is low.

On the other hand, a pervaporation membrane formed of a ZSM-5 type zeolite or a silicalite is a pervaporation membrane having the same function of allowing the organic substances to permeate as the pervaporation membrane according to the present invention. However, the membrane formed of the above material is a porous membrane differing from the highly dense membrane of the present invention. In the porous membrane, a blockage caused by the impurity such as the inorganic salt and the polymer is likely to occur. Thus, there is a problem in that the porous membrane cannot avoid the deterioration of the separation property due to the blockage.

Further, since a pervaporation membrane formed of polydimethylsiloxane is an organic material permeating membrane as well as a highly dense membrane, the aforementioned blockage is unlikely to occur. However, there is a problem in that the pervaporation membrane formed of the polydimethylsiloxane has a low separation property for the organic substances.

In contrast, since the pervaporation membrane 1 according to the present invention includes the support body 11 and the highly dense enveloping layer 12 formed of the polyamide-containing resin, it is possible to highly strike the balance between the separation property for the organic substances and the durability even if the treatment target liquid contains the impurity such as the inorganic salt and the polymer. With this pervaporation membrane 1, it is possible to continuously carry out a separating and concentrating treatment to the treatment target liquid having a low concentration and containing the impurity over the long term with suppressing exchange frequency of the pervaporation membrane 1. As a result, it is possible to efficiently obtain the phenol material which is industrially applicable and the treatment target liquid to which the disposal treatment and the detoxifying treatment can be applied at low cost.

Especially, the pervaporation membrane 1 according to the present invention is also useful for treating the treatment target liquid containing the inorganic ion in the amount of equal to or more than 0.1 percent by mass because the pervaporation membrane 1 can be used for the separating and concentrating treatment over the long term. Namely, in the case where the treatment target liquid contains the inorganic ion, a separation property of a traditional pervaporation membrane significantly deteriorates and the traditional pervaporation membrane deteriorates in a short time. As a result, there is a problem in that an efficiency of the separating and concentrating treatment significantly deteriorates. In contrast, since the pervaporation membrane 1 according to the present invention includes the support body 11 and the enveloping layer 12, the pervaporation membrane 1 can reliably solve these problems. As a result, it is possible to obtain the pervaporation membrane 1 which can efficiently carry out the separating and concentrating treatment without preliminarily carrying out a treatment such as a removing treatment of removing the inorganic ion from the treatment target liquid or the like.

In addition to the above treatment target liquid containing the inorganic ion, the traditional pervaporation membrane cannot be applied to strong basic treatment target liquid having a pH value more than 7 or 8 over the long term. In contrast, the pervaporation membrane 1 according to the present invention can efficiently carry out the separating and concentrating treatment to such strong basic treatment target liquid over the long term.

(Filler)

The pervaporation membrane 1 may contain filler as needed. The filler means particles dispersed in the polyamide-containing resin and act so as to improve various properties such as the mechanical property of the pervaporation membrane 1. Examples of a shape of the filler include a spherical shape, a plate-like shape (a scale-like shape) and a needle-like shape.

An average particle size of the filler is preferably in the range of about 0.01 to 20 μm, and more preferably in the range of about 0.05 to 10 μm. Regarding the average particle size of the filler, a particle size at a 50% accumulation point on a mass basis in a particle size distribution measured by a leaser diffractometry particle size measuring method

is defined as the average particle size of the filler.

Examples of a constituent material for the filler include an inorganic material such as silica, alumina, titania, zirconia, mica, clay and zeolite and an organic material such as a phenol resin, an acrylic resin, an acrylonitrile resin, a polyurethane, a polyvinyl chloride, a polystyrene and a polyamide. Among them, the zeolite is preferably used and a hydrophobic zeolite is more preferably used from the viewpoint of the separation efficiency for the phenols.

Although an occupancy rate of the filler in the pervaporation membrane 1 is not particularly limited to a specific value, the occupancy rate of the filler is preferably less than the occupancy rate of the support body 11. In particular, the occupancy rate of the filler in the pervaporation membrane 1 is preferably in the range of about 2 to 40 percent by volume, and more preferably in the range of about 10 to 35 percent by volume. By setting the occupancy rate of the filler to fall within the above range, it is possible to further improve the mechanical property of the pervaporation membrane 1 without deteriorating the separation property due to the polyamide-containing resin.

A surface treatment for improving an adhesion property with respect to the polyamide-containing resin may be applied to a surface of the filler as needed. Examples of such a surface treatment include a coupling agent treatment and a plasma treatment.

(Electron beam irradiation treatment)

An electron beam irradiation treatment may be applied to the pervaporation membrane 1 as needed. The electron beam irradiation treatment is carried out by irradiating the enveloping layer 12 or the like with the electron beam to cause a property modification. By irradiating the enveloping layer 12 formed of the aforementioned polyamide-containing resin with the electron beam, the property modification occurs at the surface as well as at an internal area of the enveloping layer 12 due to energy of the electron beam. This makes it possible to improve a cross-link density of the polymer in the enveloping layer 12, and thereby improving the mechanical strength. As a result, the durability of the pervaporation membrane 1 is improved. For example, the deterioration of the pervaporation membrane 1 becomes unlikely to occur even if the pervaporation membrane 1 swells. Further, due to the electron beam irradiation treatment, it is unlikely to occur that a chemical bond or a functional group such as an amide bond providing the affinity with respect to the organic substances disappears or is modified. Thus, whereas the mechanical strength is improved as described above, the separation property for the organic substances little deteriorates. Further, since the separation property for the organic substances is unlikely to deteriorate and the pervaporation membrane 1 is unlikely to be broken even in the case where the pervaporation membrane 1 is used for the process of the pervaporation over the long term, the pervaporation membrane 1 is especially useful. Furthermore, the adhesion property with respect to the treatment target liquid is improved because a functional group is introduced to the surface of the membrane due to the irradiation of the electron beam. As a result, the contact property between the organic substances and the membrane is also improved, and thereby improving the separation property for the organic substances. Therefore, by applying the electron beam irradiation treatment to the enveloping layer 12 formed of the polyamide-containing resin, it is possible to obtain the pervaporation membrane 1 highly striking the balance between the separation property for the organic substances and the durability.

The electron beam is one kind of radiation and a bundle of electrons obtained by accelerating the electrons with an accelerator or the like. Since the electron beam generally has a high directive property, it is possible to uniform an accumulated irradiation amount over a treated surface by scanning the electron beam over the membrane formed of the polyamide-containing resin in the electron beam irradiation treatment. A scanning pattern of the electron beam is not particularly limited to a specific pattern and the accumulated irradiation amount may not be constant.

The accumulated irradiation amount (absorbed dose) of the electron beam is not particularly limited to a specific value, but is preferably in the range of about 25 to 300 kGy, and more preferably in the range of about 30 to 200 kGy. By setting the accumulated irradiation amount to fall within the above range, it is possible to improve the mechanical strength of the enveloping layer 12 with reliably suppressing the disappearance or the modification of the chemical bond or the functional group providing the affinity with respect to the organic substances. Further, it is possible to obtain the pervaporation membrane 1 which can keep these effects over the long term.

An accelerating voltage for accelerating the electrons to obtain the electron beam is preferably in the range of about 10 to 200 kV, and more preferably in the range of about 20 to 150 kV. The accelerating voltage affects a depth of penetration of the electron beam with respect to the enveloping layer 12. Thus, by setting the accelerating voltage to fall within the above range, it is possible to apply the necessary and sufficient treatment to an entire of the enveloping layer in a thickness direction thereof. As a result, it is possible to more highly strike the balance between the separation property and the durability.

In the case where the polyamide-containing resin is the copolymer containing the polyamide segment and the polyether segment, the electron beam irradiation treatment can especially provide a good effect for striking the balance between the separation property and the durability of the membrane. Namely, since the polyether segment has high permeability with respect to the organic substances and a membrane structure of the polyether segment is easily and appropriately strengthened by the electron beam irradiation treatment. Thus, it is possible to obtain the pervaporation

membrane 1 which can simultaneously provide two effects of keeping the property of the polyether segment (that is the high permeability with respect to the organic substances) and improving the durability with respect to the swelling of the organic substance. The two effects cannot be simultaneously obtained by only improving the permeability with respect to the organic substances.

Further, depending on the condition of the electron beam irradiation treatment and the composition of the polyamide-containing resin, it is possible to form a concave-convex shape on an irradiated surface. With this treatment, it is possible to increase a contact area between the surface and the treatment target liquid, and thereby further improving the treatment efficiency of the pervaporation method. Thus, it is preferable that the pervaporation membrane 1 is provided in the pervaporation membrane module 120 so that the irradiated surface irradiated by the electron beam faces to the supply side space 122.

Further, it is preferable that the electron beam irradiation treatment is carried out in the presence of inert gas. By carrying out the electron beam irradiation treatment in the presence of the inert gas, it is possible to suppress oxygen from affecting an electron beam irradiated part, and thereby preventing the disappearance of the chemical bond or the functional group providing the affinity with respect to the organic substances. In this case, an oxygen density is preferably set to be equal to or less than 500 ppm by mass ratio. Examples of the inert gas include nitrogen gas and argon gas.

Hereinabove, although the pervaporation membrane 1 is described, the pervaporation membrane 1 of the present invention may be a laminated membrane obtained by laminating a plurality of pervaporation membranes 1 described above, a laminated membrane obtained by laminating the pervaporation membrane 1 and other membranes or the like. In these cases, these membranes can also provide the same effects as the aforementioned pervaporation membrane 1.

Subsequently, description will be given to a producing method for the pervaporation membrane 1. Although the pervaporation membrane 1 can take a various forms as described above, description will be given to a case where the pervaporation membrane 1 is the flat membrane.

The pervaporation membrane 1 in the form of the flat membrane can be produced by, for example, melting a raw material at a temperature in the range of about 180 to 230°C and then forming a resulting molten material into the form of the flat membrane with a forming method such as an extrusion method, a mold injection method and a press method. The raw material may be diluted with a solvent or the like as needed.

Further, a surface treatment other than the electron beam irradiation treatment may be applied to the surface of the pervaporation membrane 1 as needed. Examples of such a surface treatment include a corona discharge treatment, an arc discharge treatment, an irradiation treatment of excimer light, a plasma treatment, an etching treatment and a coating treatment. Further, examples of the coating treatment include a treatment of coating the surface of the pervaporation membrane 1 to introduce a functional group having high affinity with respect to the organic substances to be separated.

<Phenol concentrating method>

Subsequently, description will be given to the embodiment of the phenol concentrating method according to the present invention. Although the aforementioned pervaporation membrane of the present invention targets liquid containing a wide variety of organic substances as a treatment target (treatment target liquid), description will be given to the case where the phenol aqueous solution is the treatment target liquid, hereinafter.

Fig. 4 (a) is a process chart for the phenol concentrating method according to this embodiment. Fig. 4(b) is a view for explaining changing of a treated material in the phenol concentrating method according to this embodiment. The phenol concentrating method shown in Fig. 4 includes a pretreatment process S1 for pretreating treatment target liquid A1, a pervaporation process S2 for applying the pervaporation method using the pervaporation membrane 1 to pretreated treatment target liquid A2 to preferentially vaporize phenols in the treatment target liquid A2, and a condensation process for condensing a permeated material A3 in a gaseous state to obtain a condensed material A4 in a liquid state or a solid state. By carrying out these processes, it is possible to obtain the condensed material A4 which is a concentrated material of the phenols. Hereinafter, each process is described in detail.

[1] Pretreatment process S1

In the pretreatment process S1, the sedimentation treatment with the flocculant, the filtration treatment, the reverse osmosis membrane treatment, the azeotropic treatment, the distillation treatment or the like as described above is carried out to remove foreign substances in the treatment target liquid A1 and carry out a volume reduction of the treatment target liquid A1. Among them, in the reverse osmosis membrane treatment, water contained in the treatment target liquid A1 is removed by using a reverse osmosis membrane (RO membrane) to improve a phenol concentration. As a result, it is possible to carry out the volume reduction of the treatment target liquid A1. The treatment target liquid A2 is obtained through such a pretreatment.

The pretreatment process S1 is carried out as needed. Depending on an amount of the treatment target liquid A1, a composition and a concentration of the organic substances contained in the treatment target liquid A1 or the like, the pretreatment process S1 may be omitted.

[2] Pervaporation process S2

In the pervaporation process S2, the treatment target liquid A2 is supplied into the supply side space 122 of the pervaporation membrane module 120 shown in Fig. 2. At this time, the permeation side space 123 of the pervaporation membrane module 120 is depressurized. With this process, the phenols in the treatment target liquid A2 preferentially permeate across the pervaporation membrane 1. At this time, the phenols and the water transit into the gaseous state and permeate across the pervaporation membrane 1. As a result, the permeated material A3 in the gaseous state is obtained.
A pressure in the permeation side space 123 is preferably in the range of about 10 to 7000 Pa, and more preferably in the range of about 50 to 5000 Pa. By setting the pressure in the permeation side space 123 to fall within the above range, it is possible to efficiently concentrate the phenols.
A temperature of the treatment target liquid A2 is preferably in the range of about 30 to 95°C, and more preferably in the range of 40 to 80°C. By setting the temperature of the treatment target liquid A2 to fall within the above range, it is possible to improve a treatment efficiency of the treatment target liquid A2.
The pervaporation membrane 1 can separate and concentrate the organic substances from the treatment target liquid containing the organic substances. Especially, the pervaporation membrane 1 is useful for separating and concentrating the phenols. This is resulted from a relationship between a molecular structure of the phenol and the composition of the pervaporation membrane 1. In particular, it is considered as the reason of the above result that the phenol has high affinity with respect to a structure in the polyamide. Further, the pervaporation membrane 1 has a high separation property for the phenols and it can suppress the deterioration of the membrane caused by the osmosis of the phenols to a minimum. It is considered that this is because the pervaporation membrane 1 includes the enveloping layer 12 formed of the polyamide-containing resin and the support body 11 supporting the enveloping layer 12 and the support body 11 improves the durability of the membrane without deteriorating the separation property of the enveloping layer 12.
Thus, according to the phenol concentrating method of this embodiment, it is possible to efficiently separate and concentrate the phenols over the long term. Further, since the deterioration of the pervaporation membrane 1 caused by the pervaporation of the phenols can be suppressed to a minimum, it is possible to obtain the pervaporation membrane 1 which can keep carrying out the separation treatment of the phenols over the long term without exchanging the pervaporation membrane 1 or with suppressing the exchange frequency of the pervaporation membrane 1 to a minimum.

[3] Condensation process S3

In the condensation process S3, the permeated material A3 in the gaseous state is condensed. With this process, the permeated material A3 devolatilizes and the condensed material A4 in the liquid state is obtained. Alternatively, depending on a condensation condition, the condensed material A4 in the solid state is obtained.
A general condensing method can be used for condensing the permeated material A3. For example, the permeated material A3 may be pressured (to atmospheric pressure) or cooled. For cooling the permeated material A3, a wide variety of cooling apparatus or coolant such as liquid nitrogen may be used.
The condensation process S3 may be carried out as needed. For example, the condensation process S3 may be replaced with a process for preferentially depositing the phenols in the solid state or the liquid state from the permeated material A3 in the gaseous state. This deposition utilizes differences of temperatures or pressures, which cause state changes of the phenol and the water, between the phenol and the water. Namely, when state diagrams of the phenol and the water are compared with each other, temperatures and pressures passing through a vapor-liquid equilibrium curve have differences between the phenol and the water. By utilizing these differences between the temperatures and the pressures of the phenols and the water, it is possible to preferentially change a state of the phenols in the permeated material A3 in the gaseous state to deposit the phenols. For example, by changing at least one of the temperature and the pressure so that at least one of the temperature and the pressure gets across only a solid-gaseous equilibrium curve of the phenol (in other words, so that at least one of the temperature and the pressure does not get across a solid-gaseous equilibrium curve of the water), it is possible to preferentially deposit the phenols in the solid state from the permeated material A3 in the gaseous state. Further, the same manner can be applied to the case of depositing the phenols in the liquid state.
On the other hand, since the water remains in the gaseous state after the deposition of the phenols, the states of the phenols and the water become different from each other. Thus, it is possible to separate and recover the phenols in the solid state at a high yield. By recovering the phenols in the solid state in this manner, it is possible to obtain the phenols having a higher concentration compared with the case where the condensed material A4 is obtained. As a

result, handling of the recovered phenols becomes easier and the industrial application thereof is further facilitated.

Although the pervaporation membrane and the phenol concentrating method of the present invention have been described, the present invention is not limited thereto.

For example, additional processes for arbitrary objects may be added to the phenol concentrating method. Further, the phenol concentrating method can be applied for concentrating the organic substances other than phenols.

## EXAMPLES

Next, description will be given to examples of the present invention.

1. Concentrating phenol aqueous solution

A pervaporation membrane was formed and phenol aqueous solution was concentrated as explained in examples and comparative examples. Conditions of the obtained pervaporation membranes of the examples and the comparative examples are shown in tables 1 and 2.

(Example 1)

(1) Forming flat membrane

First, a membrane material M1 was heat-melted at a temperature of 200°C and then a resulting molten material was formed into the form of a flat membrane with an extrusion method to obtain a flat membrane. The membrane material M1 was a block copolymer containing segments shown in the table 1.

(2) Laminating support body and flat membrane

Subsequently, a glass cloth ("WEA116E" made by Nitto Boseki Co., Ltd., having a mass of 105 g/$m^2$) was prepared as a support body. The support body was laminated on the aforementioned flat membrane and set into a press machine. In the press machine, silicone rubber sheets (having a thickness of 500 μm) were used as upper and lower patch plates contacting with a pressing object. By pressing the support body and the flat membrane at a pressure of 50 kg/$cm^2$ for 5 minutes with the press machine to pressure-bond the support body and the flat membrane, a pervaporation membrane was obtained. An average thickness of the obtained pervaporation membrane was 130 μm. The average thickness of the pervaporation membrane was measured with a contact-type digimatic indicator. An occupancy rate of a polyamide-containing resin in the pervaporation membrane was 36 percent by volume.

(3) Pervaporation treatment for phenol aqueous solution

Subsequently, the produced pervaporation membrane was attached to the pervaporation membrane module shown in Fig. 1. Then, phenol aqueous solution was supplied into the supply side space of the pervaporation membrane module. The supplied phenol aqueous solution was aqueous solution (having a pH value of 8.5) having a phenol concentration of 2 percent by mass and an inorganic ionic impurity concentration of 1 percent by mass. A temperature of the phenol aqueous solution was set to 60°C.

On the other hand, the permeation side space of the pervaporation membrane module was depressurized to a pressure of 133 Pa. Then, a gaseous phase component permeating across the pervaporation membrane was devolatilized with a glass trap cooled by liquid nitrogen to recover it. With this process, concentrated liquid was obtained. Further, a circulating conduit was provided so that the discharged phenol aqueous which did not permeate across the pervaporation membrane in the pervaporation membrane module was transferred back to the supply side space again.

(Example 2)

A pervaporation membrane of example 2 was obtained and the pervaporation treatment was applied to the phenol aqueous solution in the same manner as the example 1 except that the support body was changed to the following material.

A polyester non-woven cloth ("MF90" made by Japan Vilene Company, Ltd., having a mass of 90 g/$m^2$) was used as the support body. An average thickness of the obtained pervaporation membrane was 130 μm. An occupancy rate of the polyamide-containing resin in the pervaporation membrane was 46 percent by volume.

(Example 3)

The pervaporation treatment was applied to phenol aqueous solution of example 3 in the same manner as the example 1 except that aqueous solution (having a pH value of 8.0) having a phenol concentration of 7 percent by mass and an inorganic ionic impurity concentration of 1 percent by mass was used as the phenol aqueous solution. Further, the occupancy rate (volume fraction) of the support body was changed to a value shown in the table 1.

(Example 4)

A pervaporation membrane of example 4 was obtained and the pervaporation treatment was applied to the phenol aqueous solution in the same manner as the example 1 except that the membrane material M1 was changed to a membrane material M3 and the occupancy rate (volume fraction) of the support body was changed to a value shown in the table 1. The membrane material M3 was a block copolymer containing segments shown in the table 1.

(Example 5)

A pervaporation membrane of example 5 was obtained and the pervaporation treatment was applied to the phenol aqueous solution in the same manner as the example 1 except that the membrane material M1 was changed to a membrane material M4 and the occupancy rate (volume fraction) of the support body was changed to a value shown in the table 1. The membrane material M4 was a block copolymer containing segments shown in the table 1.

(Example 6)

A pervaporation membrane of example 6 was obtained and the pervaporation treatment was applied to the phenol aqueous solution in the same manner as the example 1 except that the membrane material M1 was changed to a membrane material M5 and the occupancy rate (volume fraction) of the support body was changed to a value shown in the table 1. The membrane material M5 was a block copolymer containing segments shown in the table 1.

(Example 7)

A pervaporation membrane of example 7 was obtained and the pervaporation treatment was applied to the phenol aqueous solution in the same manner as the example 1 except that the membrane material M1 was changed to a membrane material M6 and the occupancy rate (volume fraction) of the support body was changed to a value shown in the table 1. The membrane material M6 was a block copolymer containing segments shown in the table 1.

(Example 8)

A pervaporation membrane of example 8 was obtained and the pervaporation treatment was applied to the phenol aqueous solution in the same manner as the example 1 except that the membrane material M1 was changed to a membrane material M7 and the occupancy rate (volume fraction) of the support body was changed to a value shown in the table 1. The membrane material M7 was a block copolymer containing segments shown in the table 1.

(Example 9)

A pervaporation membrane of example 9 was obtained and the pervaporation treatment was applied to the phenol aqueous solution in the same manner as the example 1 except that the membrane material M1 was changed to a membrane material M8 and the occupancy rate (volume fraction) of the support body was changed to a value shown in the table 1. The membrane material M8 was a block copolymer containing segments shown in the table 1.

(Example 10)

A pervaporation membrane of example 10 was obtained and the pervaporation treatment was applied to the phenol aqueous solution in the same manner as the example 1 except that the membrane material M1 was changed to a membrane material M9 and the occupancy rate (volume fraction) of the support body was changed to a value shown in the table 1. The membrane material M9 was a block copolymer containing segments shown in the table 1.

(Example 11)

A pervaporation membrane of example 11 was obtained and the pervaporation treatment was applied to the phenol aqueous solution in the same manner as the example 1 except that the membrane material M1 was changed to a membrane material M10 and the occupancy rate (volume fraction) of the support body was changed to a value shown in the table 1. The membrane material M10 was a block copolymer containing segments shown in the table 1.

(Example 12)

A pervaporation membrane of example 12 was obtained and the pervaporation treatment was applied to the phenol aqueous solution in the same manner as the example 1 except that the occupancy rate (volume fraction) of the support body was changed to a value shown in the table 1.

(Example 13)

A pervaporation membrane of example 13 was obtained and the pervaporation treatment was applied to the phenol aqueous solution in the same manner as the example 1 except that the occupancy rate (volume fraction) of the support body was changed to a value shown in the table 1.

(Example 14)

A pervaporation membrane of example 14 was obtained and the pervaporation treatment was applied to the phenol aqueous solution in the same manner as the example 1 except that the membrane material M1 was changed to a membrane material M11. The membrane material M11 was a blend material of nylon 12 (50 mole%) and polyethylene (50 mole%).

(Example 15)

A pervaporation membrane of example 15 was obtained and the pervaporation treatment was applied to the phenol aqueous solution in the same manner as the example 2 except that the membrane material M1 was changed to a membrane material M12 and the occupancy rate (volume fraction) of the support body was changed. The membrane material M12 was a material obtained by adding filler of a hydrophobic zeolite (having an average particle size of 3 μm) into the membrane material M1. A volume fraction of the filler in the pervaporation membrane was 10%.

(Example 16)

A pervaporation membrane of example 16 was obtained and the pervaporation treatment was applied to the phenol aqueous solution in the same manner as the example 2 except that the membrane material M1 was changed to a membrane material M13 and the occupancy rate (volume fraction) of the support body was changed. The membrane material M13 was a material obtained by adding filler of a hydrophobic zeolite (having an average particle size of 3 μm) into the membrane material M1. A volume fraction of the filler in the pervaporation membrane was 5%.

(Example 17)

A pervaporation membrane of example 17 was obtained and the pervaporation treatment was applied to the phenol aqueous solution in the same manner as the example 2 except that the membrane material M1 was changed to a membrane material M14 and the occupancy rate (volume fraction) of the support body was changed. The membrane material M14 was a material obtained by adding filler of a hydrophobic zeolite (having an average particle size of 3 μm) into the membrane material M1. A volume fraction of the filler in the pervaporation membrane was 2%.

(Example 18)

The pervaporation treatment was applied to the phenol aqueous solution in the same manner as the example 1 except that the pervaporation membrane produced in the same manner as the example 1 was irradiated with electron beam with the following conditions.

<Conditions for electron beam irradiation treatment>

Accelerating voltage: 150 kV
Absorbed dose: 100 kGy

(Example 19)

A pervaporation membrane of example 19 was obtained in the same manner as the example 1 except that plates (formed of a stainless steel) to which a mirror-like finishing was applied were used as the patch plates of the press machine. An average thickness of the obtained pervaporation membrane was 130 μm.

(Comparative example 1)

The pervaporation treatment was applied to the phenol aqueous solution in the same manner as the example 1 except that the support body was omitted and a flat membrane formed of the polyamide-containing resin was used as the pervaporation membrane.

(Comparative example 2)

The pervaporation treatment was applied to the phenol aqueous solution in the same manner as the example 1 except that the support body was omitted, a flat membrane formed of the polyamide-containing resin was used as the pervaporation membrane and aqueous solution (having a pH value of 8.0) having a phenol concentration of 7 percent by mass and an inorganic ionic impurity concentration of 1 percent by mass was used as the phenol aqueous solution.

(Comparative example 3)

The pervaporation treatment was applied to the phenol aqueous solution in the same manner as the example 1 except that a flat membrane (having an average thickness of 100 μm) formed of polydimethylsiloxane (PDMS) was used as the pervaporation membrane.

(Comparative example 4)

The pervaporation treatment was applied to the phenol aqueous solution in the same manner as the example 1 except that a flat membrane formed of polydimethylsiloxane (PDMS), in which the support body used in the example 1 was embedded so that an entire average thickness became 130 μm, was used as the pervaporation membrane.

2. Evaluation of physical properties of polyamide-containing resin

2.1 Shore D hardness

A Shore D hardness of a hardened material of the polyamide-containing resin used in each example and comparative example was measured. The Shore D hardness of the polyamide-containing resin was measured with a measuring method defined by ISO 868. Each measured Shore D hardness is shown in the tables 1 and 2.

2.2 Bending elastic modulus

A bending elastic modulus of the hardened material of the polyamide-containing resin used in each example and comparative example was measured. The bending elastic modulus of the polyamide-containing resin was measured with a measuring method defined by ISO 178. In the measuring method, a thickness of a test piece was set to 100 μm, a width of the test piece was set to 10 mm, and a distance between support points was set to 50 mm. Each measured bending elastic modulus is shown in the tables 1 and 2.

2.3 Melting point

A melting point of the hardened material of the polyamide-containing resin used in each example and comparative example was measured. The melting point of the polyamide-containing resin was measured with a measuring

method defined by ASTM D3418. Each measured melting point is shown in the tables 1 and 2.

2.4 Heat distortion temperature

A heat distortion temperature of the hardened material of the polyamide-containing resin used in each example and comparative example was measured. The heat distortion temperature of the polyamide-containing resin was measured with a measuring method defined by ISO 75. In the measuring method, pressure added to a test piece was set to 0.46 MPa. Each measured heat distortion temperature is shown in the tables 1 and 2.

3. Evaluation of separation property of pervaporation membrane

A phenol concentration of the concentrated liquid obtained by the pervaporation treatment for 24 hours in each example and comparative example was measured. At this time, a separation coefficient and a total permeation flux were also calculated. Measurement results and calculation results are shown in the tables 1 and 2. In the pervaporation treatment for 24 hours, the phenol aqueous solution (that is the treatment target liquid) was exchanged every 6 hours.

A phenol concentration before the pervaporation treatment (hereinafter, referred to as "supply phenol concentration") and a phenol concentration after the pervaporation treatment (hereinafter, referred to as "permeation phenol concentration") were measured with a capillary gas chromatography system "GC-2014" made by Shimadzu Corporation.

A water concentration before the pervaporation treatment (hereinafter, referred to as "supply water concentration") and a water concentration after pervaporation treatment (hereinafter, referred to as "permeation water concentration") were also measured. The separation coefficient was calculated with the following relational expression.

[0149] Separation coefficient = (Permeation phenol concentration / permeation water concentration) / (supply phenol concentration / supply water concentration)

The total permeation flux was calculated with the following relational expression.

Total permeation flux = Permeating amount / effective area of pervaporation membrane

4. Evaluation of durability of pervaporation membrane

An exterior view of each pervaporation membrane used for the pervaporation treatment for 24 hours was visually observed. The obtained observing results were evaluated according to the following criteria. The evaluation results are shown in the table 1 and 2.

<Evaluation criteria>

A: No changes (creases, deflections or the like) in the exterior view of the pervaporation membrane were confirmed.
B: Changes (creases, deflections or the like) were confirmed in an area equal to or less than 10% area ratio of the pervaporation membrane.
C: Changes (creases, deflections or the like) were confirmed in an area more than 10% area ratio of the pervaporation membrane.
D: Changes (creases, deflections or the like) were confirmed in an area more than 50% area ratio of the pervaporation membrane, or a breaking occurred in the pervaporation membrane.

Further, the pervaporation treatment for 96 hours was first applied to the pervaporation membrane obtained in each example and comparative example and then the pervaporation treatment for 384 hours was applied to the pervaporation membrane. After each pervaporation treatment for 96 hours and 384 hours, the external view of the pervaporation membrane was visually observed and the observing results were evaluated according to the above criteria. The evaluation results are shown in the table 1 and 2.

Table 1

| | | | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 |
|---|---|---|---|---|---|---|---|---|---|---|---|---|---|---|---|---|
| Pervaporation membrane | Membrane material | | — | M1 | M1 | M1 | M3 | M4 | M5 | M6 | M7 | M8 | M9 | M10 | M1 | M1 |
| | Composition of polyamide-containing resin | Polyamide segment | mole% | 40 | 40 | 40 | 88 | 74 | 62 | 49 | 34 | 13 | 40 | 40 | 40 | 40 |
| | | Polyether segment | mole% | 60 | 60 | 60 | 12 | 26 | 38 | 51 | 66 | 87 | | 57 | 60 | 60 |
| | | Polyester segment | mole% | | | | | | | | | | 60 | 3 | | |
| | | Blend | mole% | | | | | | | | | | | | | |
| | Physical properties of polyamide-containing resin | Shore D hardness | — | 42 | 42 | 42 | 72 | 69 | 63 | 55 | 33 | 25 | — | — | 42 | 42 |
| | | Bending elastic modulus | Mpa | 84 | 84 | 84 | 730 | 390 | 290 | 160 | 25 | 15 | — | — | 84 | 84 |
| | | Melting point | °C | 160 | 160 | 160 | 174 | 172 | 169 | 159 | 144 | 134 | — | — | 160 | 160 |
| | | Heat distortion temperature | °C | 52 | 52 | 52 | 106 | 99 | 90 | 66 | 46 | 42 | — | — | 52 | 52 |
| | Support body | Glass fiber | volume% | 64 | | 61 | 36 | 42 | 60 | 59 | 70 | 75 | 44 | 45 | 26 | 84 |
| | | Polyester non-woven cloth | volume% | | 54 | | | | | | | | | | | |
| | Filler | Hydrophobic zeolite | volume% | | | | | | | | | | | | | |
| | Surface roughness | Arithmetic mean roughness Ra | μm | 7.6 | 5.8 | 7.8 | 5.4 | 5.3 | 6.6 | 7.6 | 8.8 | 10.1 | 6.9 | 7.5 | 7.1 | 6.4 |
| | Electron beam irradiation treatment | | — | None | None | None | None | None | None | None | None | None | None | None | None | None |
| | Pervaporation treatment time | | h | 24/96/384 | 24/96/384 | 24/96/384 | 24/96/384 | 24/96/384 | 24/96/384 | 24/96/384 | 24/96/384 | 24/96/384 | 24/96/384 | 24/96/384 | 24/96/384 | 24/96/384 |
| Evaluation results | Supply phenol concentration | | mass% | 2 | 2 | 7 | 2 | 2 | 2 | 2 | 2 | 2 | 2 | 2 | 2 | 2 |
| | Permeation phenol concentration | | mass% | 50 | 47 | 74 | 48 | 45 | 49 | 49 | 46 | 45 | 40 | 47 | 48 | 47 |
| | Sepration coefficient | | — | 49 | 43 | 38 | 45 | 40 | 47 | 47 | 42 | 40 | 33 | 43 | 45 | 43 |
| | Total permeation flux | | $g/m^2h$ | 31 | 36 | 105 | 8 | 12 | 17 | 23 | 42 | 63 | 21 | 34 | 35 | 33 |
| | Total permeation flux / polymer volume fraction | | $g/m^2h$ | 86 | 78 | 269 | 13 | 21 | 43 | 56 | 140 | 252 | 38 | 62 | 47 | 206 |
| | Swelling rate | | % | 10 | 14 | 41 | 4 | 4 | 5 | 8 | 17 | 28 | 9 | 12 | 18 | 9 |
| | Form change of sepraratingmembrane after 24 hours treatment | | — | A | A | B | A | A | A | A | B | B | A | A | A | A |
| | Form change of sepraratingmembrane after 96 hours treatment | | — | B | A | B | A | A | A | A | B | B | B | A | A | A |
| | Form change of sepraratingmembrane after 384 hours treatment | | — | B | B | B | A | A | A | B | B | C | C | B | B | B |

Table 2

| | | | | Ex. 14 | Ex. 15 | Ex. 16 | Ex. 17 | Ex. 18 | Ex. 19 | CEx. 1 | CEx. 2 | CEx. 3 | CEx. 4 |
|---|---|---|---|---|---|---|---|---|---|---|---|---|---|
| Pervaporation membrane | Membrane material | | – | M11 | M12 | M13 | M14 | M1 | M1 | M1 | M1 | PDMS | PDMS |
| | Composition of polyamide-containing resin | Polyamide segment | mole% | | 40 | 40 | 40 | 40 | 40 | 40 | 40 | | |
| | | Polyether segment | mole% | | 60 | 60 | 60 | 60 | 60 | 60 | 60 | | |
| | | Polyester segment | mole% | | | | | | | | | | |
| | | Blend | mole% | 100 | | | | | | | | | |
| | Physical properties of polyamide-containing resin | Shore D hardness | – | – | 42 | 42 | 42 | 42 | 42 | 42 | 42 | – | – |
| | | Bending elastic modulus | Mpa | – | 84 | 84 | 84 | 84 | 84 | 84 | 84 | – | – |
| | | Melting point | ℃ | – | 160 | 160 | 160 | 160 | 160 | 160 | 160 | – | – |
| | | Heat distortion temperature | ℃ | – | 52 | 52 | 52 | 52 | 52 | 52 | 52 | – | – |
| | Support body | Glass fiber | volume% | 63 | | | | 64 | 61 | | | | 50 |
| | | Polyester non-woven cloth | volume% | | 40 | 60 | 50 | | | | | | |
| | Filler | Hydrophobic zeolite | volume% | | 10 | 5 | 2 | | | | | | |
| | Surface roughness | Arithmetic mean roughness Ra | μm | 5.8 | 4.7 | 5.5 | 5.3 | 7.6 | 0.57 | 0.46 | 0.51 | 0.21 | 8.8 |
| | Electron beam irradiation treatment | | – | None | None | None | None | Done | None | None | None | None | None |
| | Pervaporation treatment time | | h | 24/96 /384 | 24/96 /384 | 24/96 /384 | 24/96 /384 | 24/96 /384 | 24/96 /384 | 24/96 /384 | 24/96 /384 | 24/96 /384 | 24/96 /384 |
| Evaluation results | Supply phenol concentration | | mass% | 2 | 2 | 2 | 2 | 2 | 2 | 2 | 7 | 2 | 2 |
| | Permeation phenol concentration | | mass% | 42 | 46 | 44 | 56 | 58 | 48 | 33 | 0 | 7 | 5 |
| | Sepration coefficient | | – | 35 | 42 | 39 | 62 | 68 | 45 | 24 | 0 | 4 | 3 |
| | Total permeation flux | | $g/m^2h$ | 16 | 41 | 24 | 38 | 29 | 24 | 420 | – | 456 | 345 |
| | Total permeation flux / polymer volume fraction | | $g/m^2h$ | 43 | 82 | 69 | 79 | 81 | 62 | 420 | – | 456 | 690 |
| | Swelling rate | | % | 8 | 14 | 11 | 11 | 8 | 12 | 50 | 97 | 2 | 3 |
| | Form change of seprarating membrane after 24 hours treatment | | – | A | A | A | A | A | A | C | C | A | A |
| | Form change of seprarating membrane after 96 hours treatment | | – | A | A | A | A | A | A | D | D | A | A |
| | Form change of seprarating membrane after 384 hours treatment | | – | B | A | A | A | A | A | D | D | B | A |

As is clear from the tables 1 and 2, by using the pervaporation membrane obtained in each example, it is confirmed that the phenols can be efficiently concentrated. Further, the form change deteriorating the separation property

did not occur in the pervaporation membrane obtained in each example even after 24 hours. Furthermore, the form change deteriorating the separation property did not occur in the pervaporation membranes obtained in some of the examples even after 384 hours.

On the other hand, by using the pervaporation membrane obtained in each comparative example, it is confirmed that the concentration rate is low. Further, a big form change or a breaking occurs in the pervaporation membranes obtained in some of the comparative examples. Furthermore, since the total permeation flux of the pervaporation membrane obtained in each comparative example increases, it is estimated that liquid leakage occurs in the pervaporation membrane due to the big form changes or the breaking.

## INDUSTRIAL APPLICABILITY

The present invention relates to the pervaporation membrane used for concentrating the phenols in the liquid containing the phenols, the water and the inorganic ion with the pervaporation method. The pervaporation membrane includes the porous support body and the enveloping layer provided so as to envelop the support body. The enveloping layer is formed of the polyamide-containing resin. According to the present invention, it is possible to provide the pervaporation membrane having the superior durability and the phenol concentrating method which can efficiently concentrate the phenols with the pervaporation membrane. For the reasons stated above, the present invention is industrial applicable.

## DESCRIPTION OF REFERENCE NUMBER

**[0149]**

| | |
|---|---|
| 1 | Pervaporation membrane |
| 11 | Support body |
| 12 | Enveloping layer |
| 100 | Pervaporation separation apparatus |
| 110 | Treatment target liquid tank |
| 115 | Supply conduit |
| 120 | Pervaporation membrane module |
| 121 | Housing |
| 122 | Supply side space |
| 123 | Permeation side space |
| 125 | Permeation conduit |
| 126 | Discharge conduit |
| 130 | Permeated material recovering tank |
| 140 | Non-permeated material storage tank |
| 150 | Pretreatment module |
| S1 | Pretreatment process |
| S2 | Pervaporation process |
| S3 | Condensation process |
| A1, A2 | Treatment target liquid |
| A3 | Permeated material |
| A4 | Condensed material |

## Claims

1. A pervaporation membrane to be used for concentrating phenols in liquid containing the phenols, water and an inorganic ion with a pervaporation method, the pervaporation membrane comprising:

   a porous support body; and
   an enveloping layer provided so as to envelop the porous support body, the enveloping layer formed of a polyamide-containing resin.

2. The pervaporation membrane as claimed in claim 1, wherein the polyamide-containing resin is a copolymer containing a polyamide segment.

3. The pervaporation membrane as claimed in claim 2, wherein the copolymer further contains a polyether segment.

4. The pervaporation membrane as claimed in claim 3, wherein the copolymer is a block copolymer containing 10 to 90 mole% of the polyamide segment.

5. The pervaporation membrane as claimed in any one of claims 1 to 4, wherein the support body is a woven cloth or a nonwoven cloth.

6. The pervaporation membrane as claimed in claim 5, wherein the support body is constituted of a glass fiber.

7. A phenol concentrating method comprising:

   concentrating phenols in liquid containing the phenols and water with a pervaporation method using a pervaporation membrane defined in any one of claims 1 to 6.

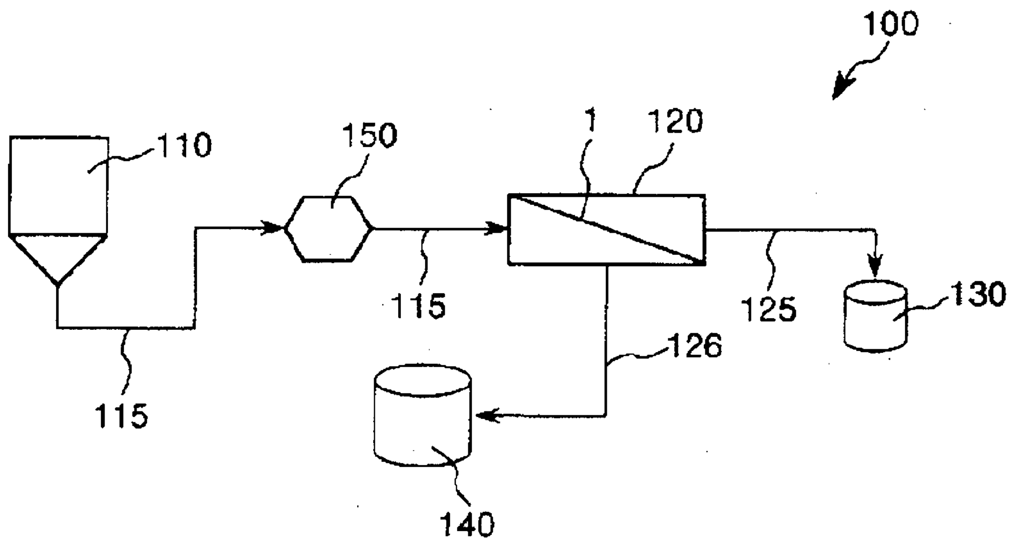


FIG.1

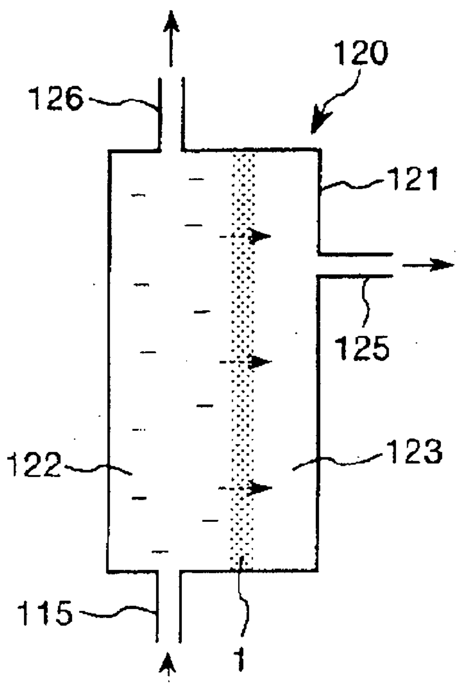


FIG.2

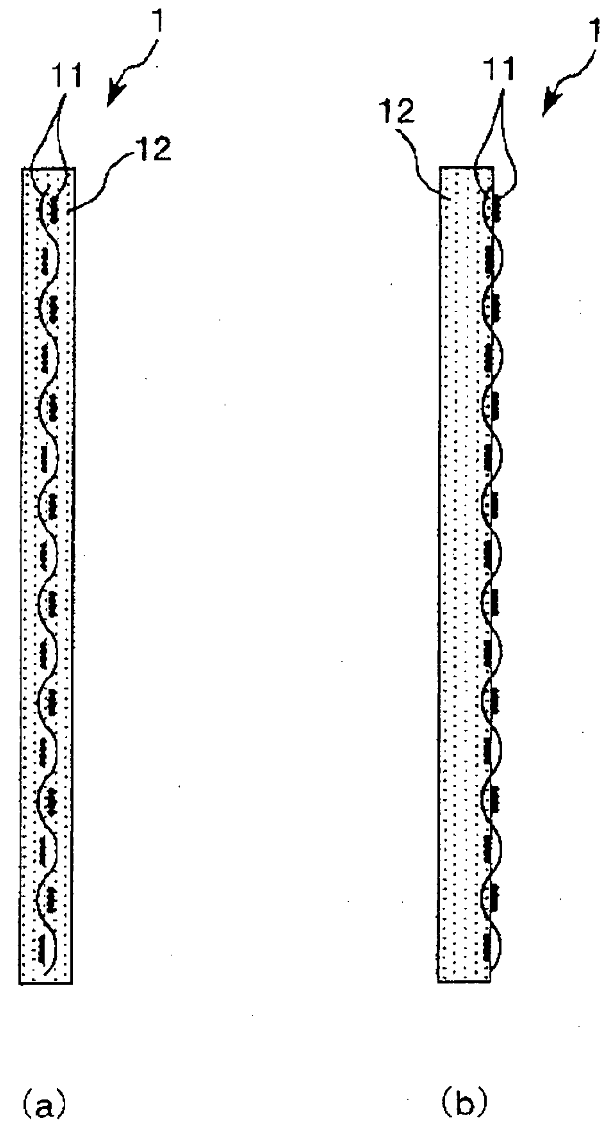


FIG.3

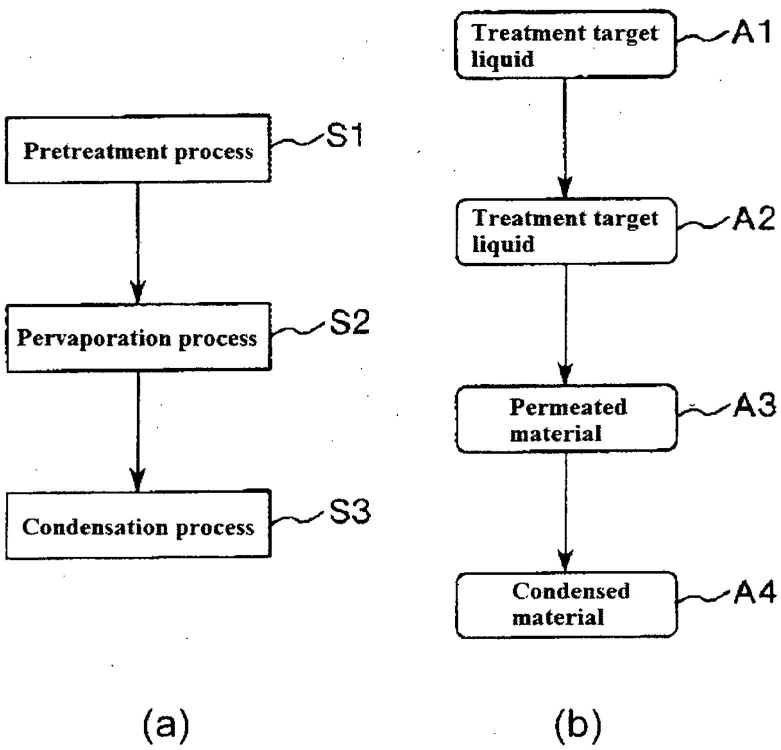
Pretreatment process
S1
Pervaporation process
S2
Condensation process
S3
(a)
Treatment target liquid
A1
Treatment target liquid
A2
Permeated material
A3
Condensed material
A4
(b)

FIG.4

**INTERNATIONAL SEARCH REPORT**

International application No. PCT/JP2014/051007

A. CLASSIFICATION OF SUBJECT MATTER

*B01D71/56*(2006.01)i, *B01D61/36*(2006.01)i, *B01D69/10*(2006.01)i, *B01D69/12*(2006.01)i, *B01D71/52*(2006.01)i, *B32B27/34*(2006.01)i, *C02F1/44*(2006.01)i, *C07C37/70*(2006.01)i, *C07C39/04*(2006.01)i

According to International Patent Classification (IPC) or to both national classification and IPC

B. FIELDS SEARCHED

Minimum documentation searched (classification system followed by classification symbols)

B01D71/56, B01D61/36, B01D69/10, B01D69/12, B01D71/52, B32B27/34, C02F1/44, C07C37/70, C07C39/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)

JSTPlus(JDreamIII), Thomson Innovation

C. DOCUMENTS CONSIDERED TO BE RELEVANT

| Category* | Citation of document, with indication, where appropriate, of the relevant passages | Relevant to claim No. |
|---|---|---|
| X<br>Y | Masakazu KONDO, "Separation of phenol in aquenous solution by pervaporation using polyether-block-amides membranes incorporating polyester substrate", Membrane, 1997, vol.22, no.6, pages 343 to 350 | 1-5,7<br>1-7 |
| Y | JP 8-24892 A (Nicca Chemical Co., Ltd.), 30 January 1996 (30.01.1996), paragraph [0003] (Family: none) | 1-7 |
| Y | JP 4-326929 A (Nitto Denko Corp.), 16 November 1992 (16.11.1992), claim 1; paragraphs [0010], [0015] to [0017] (Family: none) | 6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 March, 2014 (19.03.14) | 01 April, 2014 (01.04.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2014/051007

C (Continuation). DOCUMENTS CONSIDERED TO BE RELEVANT

| Category* | Citation of document, with indication, where appropriate, of the relevant passages | Relevant to claim No. |
|---|---|---|
| Y | JP 7-275676 A (Metallgesellschaft AG.), 24 October 1995 (24.10.1995), claim 1; paragraphs [0001], [0002] & US 5849195 A & EP 673674 A1 | 6 |
| X<br>Y | JP 8-57274 A (Nagayanagi Co., Ltd.), 05 March 1996 (05.03.1996), claims 1, 5; paragraphs [0002] to [0006], [0013], [0021], [0023] (Family: none) | 1-3,5,7<br>1-7 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H06296831 A